# EUROPÄISCHE PATENTSCHRIFT

(11) **EP 4 315 984 B1**
(45) Veröffentlichungstag und Bekanntmachung des Hinweises auf die Patenterteilung: **27.08.2025**
(21) Anmeldenummer: 22720342.9
(22) Anmeldetag: 31.03.2022
(51) Int. Cl.: H04W 36/00, H04W 4/90, H04W 88/08, H04W 84/12

(54) **VERFAHREN ZUM BETRIEB EINES TELENOTARZT-SYSTEMS UND TELENOTARZT-SYSTEM**
METHOD FOR OPERATING A TELE-EMERGENCY DOCTOR SYSTEM
PROCÉDÉ DE FONCTIONNEMENT D'UN SYSTÈME DE MÉDECIN D'URGENCE À DISTANCE

(30) Priorität: 31.03.2021 DE 102021108165
(43) Veröffentlichungstag der Anmeldung: 07.02.2024
(73) Patentinhaber: FTI Engineering Network GmbH, 15745 Wildau (DE)
(72) Erfinder: HELLMANN, Benjamin, 10587 Berlin (DE); TIMME, Sebastian, 10587 Berlin (DE)
(74) Vertreter: RavensPAT Patentanwälte Partnerschaft mbB
(86) Internationale Anmeldenummer: PCT/EP2022/058617
(87) Internationale Veröffentlichungsnummer: WO 2022/207814

(56) Entgegenhaltungen:
- EP-A1- 2 252 115
- EP-A1- 2 252 115
- US-A1- 2010 017 471
- US-A1- 2010 017 471
- US-A1- 2012 123 224
- US-A1- 2012 123 224
- US-A1- 2017 300 654
- US-A1- 2017 300 654

## Beschreibung

Die Erfindung betrifft ein Verfahren zum Betrieb eines Telenotarzt-Systems gemäß dem Oberbegriff des Anspruchs 1 sowie ein Telenotarzt-System gemäß dem Anspruch 5.

Systeme, welche die Übertragung von durch ein EKG-Gerät erfassten Vitaldaten an einen nicht am Einsatzort befindlichen Notarzt beschreiben, sind gegenwärtig in der Erprobung. Ein derartiges System ist zum Beispiel in dem Ergebnisbericht ''2020-12-18_Telenotarzt-Bayern_Ergebnisbericht" eines Projekts der IQ Medworks GmbH beschrieben. Dieser Bericht ist unter der URL https://innovationsfonds.g-ba.de/downloads/projektdokumente/36/2020-12-18_Telenotarzt-Bayern_Ergebnisbericht.pdf abrufbar.

Aus der US 2010/017471 A1 ist ein System bekannt, welches einen Defibrillator, ein Gateway-Gerät, ein Routing-Gerät und ein drahtloses Modem umfasst, wobei das System außerdem Hardware- und/oder Softwarekomponenten umfassen kann, die sich an einem entfernten Standort zum Empfangen von Daten befinden, sowie ein oder mehrere Servergeräte zum Dekodieren von Daten aus dem entfernten Standort. Ein entsprechendes Verfahren umfasst das Erfassen medizinischer Daten an einem ersten Ort, das Umwandeln der medizinischen Daten von einem analogen Signal in ein digitales Signal, das Übertragen des digitalen Signals vom ersten Ort über ein Mobilfunknetz über das Internet zu einem zweiten Ort, das Empfangen des digitalen Signals am zweiten Ort und das Rückumwandeln des digitalen Signals in ein analoges Signal zur Verarbeitung. Der erste Standort kann ein Rettungsfahrzeug sein und der zweite Standort kann eine entfernte Einrichtung, wie etwa eine Einsatzzentrale oder ein örtliches Krankenhaus sein.

Aus der EP 2 252 115 A1 ist eine Vorrichtung bekannt, die ein erstes Schnittstellenmodul umfasst, das für die Schnittstelle mit einem oder mehreren Benutzergeräten über eine WLAN-Verbindung (Wireless Local Area Network) konfiguriert ist, die ein zweites Schnittstellenmodul umfasst, das für die Schnittstelle mit einem drahtlosen Kommunikationsnetzwerk über eine WWAN-Verbindung (Wireless Wide Area Network) konfiguriert ist, und die einen Controller umfasst. Hierbei ist der Controller so konfiguriert, dass er die Verfügbarkeit von Netzbetreiber-Hotspots erkennt, wobei - sofern kein Netzbetreiber-Hotspot erkannt wird - der Verkehr zu und von den einem oder mehreren Benutzergeräten über die WWAN-Verbindung umgeleitet wird und wobei - sofern ein Netzbetreiber-Hotspot erkannt wird - der Verkehr zu und von den einem oder mehreren Benutzergeräten über den erkannten Netzbetreiber-Hotspot umgeleitet wird.

Aus der US 2017/300654 A1 ist ein Telemedizinsystem bekannt, das zur Kommunikation mit einem entfernten Operationszentrum dient, wobei Nachrichten mithilfe eines Transceivers mit Antenne gesendet/empfangen werden können. Ein Controller des Systems kann mithilfe des Transceivers eine Telemedizinsitzung mit dem Operationszentrum unter Verwendung eines Transport Morphing Protocol (TMP) herstellen, wobei es sich bei TMP um ein auf Bestätigung basierendes Benutzerdatagrammprotokoll handelt. Der Controller kann außerdem eine oder mehrere vorübergehende Netzwerkverschlechterungen maskieren, um die Ausfallsicherheit der Telemedizinsitzung zu erhöhen. Das Telemedizinsystem ermöglicht eine Echtzeitverbindung und -kommunikation zwischen dem medizinischen Personal in einem Rettungswagen und einem aufnehmenden Krankenhaus, um einem bedürftigen Patienten eine sofortige Diagnose und Behandlung zu ermöglichen.

Aus der US 2012/123224 A1 ist ein computergestützte Methode zur Bereitstellung zusammenfassender Informationen für lebensrettende Maßnahmen bekannt. Das Verfahren umfasst das Erfassen einer oder mehrerer Aktivitäten, die von einem Retter wiederholt und zyklisch an einem Opfer ausgeführt werden; das Identifizieren eines zyklischen Zeitintervalls, über das die Leistung für eine ganzzahlige Anzahl von Zyklen der einen oder mehreren Aktivitäten analysiert werden soll, und das Sammeln von Daten aus der Erfassung der einen oder mehreren Aktivitäten während des Zeitintervalls; das Generieren von Zusammenfassungsdaten aus der Analyse der einen oder mehreren Aktivitäten, die die für die eine oder mehrere Aktivitäten erfassten Daten zu einer Zusammenfassung der einen oder mehreren Aktivitäten verdichten; und das Bereitstellen einer visuellen Zusammenfassung der Leistung der einen oder mehreren Aktivitäten über das identifizierte Zeitintervall zur Anzeige für einen Benutzer.

Es ist Aufgabe der Erfindung ein Verfahren zum Betrieb eines Telenotarzt-Systems vorzuschlagen, welches eine möglichst unterbrechungsfreie telemedizinische Versorgung eines Patienten ermöglicht, welcher nach einer Erstversorgung an einem Umfallort in ein Rettungsfahrzeug verbracht wird. Weiterhin ist es Aufgabe der Erfindung, ein Telenotarzt-System vorzuschlagen, welches eine unterbrechungsfreie telemedizinische Versorgung eines Patienten ermöglicht, welcher nach einer Erstversorgung an einem Umfallort in ein Rettungsfahrzeug verbracht wird.

Diese Aufgabe wird durch die Merkmale des Anspruchs 1 bzw. 5 gelöst. In den jeweiligen Unteransprüchen sind vorteilhafte und zweckmäßige Weiterbildungen angegeben.

Das Verfahren zum Betrieb eines Telenotarzt-Systems, welches einen in einem Fahrzeug angeordneten mobilen Router, einen entfernt von dem Fahrzeug einsetzbaren portablen Router, einen Server und ein mit dem Server verbundenes Terminal für einen Notarzt umfasst, umfasst die Schritte:
- Einbinden eines WLAN-fähigen EKG-Geräts oder dergleichen in ein von dem portablen Router bereit gestelltes erstes WLAN-Netz;
- Erstversorgung eines Unfallopfers außerhalb des Fahrzeugs, wobei von dem EKG-Gerät erfasste Vitaldaten des Unfallopfers von dem portablen Router in einem Router-Betrieb aus dem ersten WLAN-Netz über ein Mobilfunknetz, in welches der portable Router eingebunden ist, an den Server übertragen werden, um diese an dem Terminal bereitzustellen;
- Verbringen des Unfallopfers nach der Erstversorgung zu dem und in das Fahrzeug und hierbei Trigger gesteuertes Umschalten des portablen Routers von seinem Router-Betrieb auf einen Repeater-Betrieb, wobei sich der portable Router hierzu als Repeater mit einem von dem mobilen Router bereit gestellten zweiten WLAN-Netz verbindet,
- Weiterleiten der von dem EKG-Gerät erfassten Vitaldaten von dem portablen Router in dem Repeater-Betrieb aus dem ersten WLAN-Netz des portablen Routers über das zweite WLAN-Netz des mobilen Routers an den mobilen Router und Weiterleiten der Vitaldaten von dem mobilen Router über ein Mobilfunknetz über eine bestehende Funkverbindung an den Server, so dass von dem EKG-Gerät erfasste Vitaldaten in einem kontinuierlichen Datenstrom an dem Terminal bereitgestellt werden. Durch ein derartiges Verfahren, bei welchem das WLAN-fähige EKG-Gerät mittels des portablen Routers wahlweise direkt über ein Mobilfunknetz oder über ein zweites WLAN-Netz mittelbar über ein Mobilfunknetz mit einem Server verbunden ist, lässt es sich vermeiden, dass zu dem WLAN-fähigen EKG-Gerät eine WLAN-Verbindung neu aufgebaut werden muss und hierdurch eine signifikante Unterbrechung der Verbindung zum Server in Kauf genommen werden muss. Diese Unterbrechung wird durch eine Verbindungsübergabe - auch Handover genannt - vermieden, bei welcher der portable Router, mit welchem das WLAN-fähige EKG-Gerät per WLAN verbunden ist, von einem Router-Betrieb in einen Repeater-Betrieb wechselt. Dies ist mit den bestehenden Protokollen und verfügbaren Routern wesentlich schneller möglich und auch besser automatisierbar als ein Abmelden des WLAN-fähigen EKG-Geräts von einem ersten WLAN-Netz und ein nachfolgendes Anmelden des WLAN-fähigen EKG-Geräts an einem zweiten WLAN-Netz.

Es ist auch vorgesehen, die Verbindung des portablen Routers über den mobilen Router und das Mobilfunknetz zu dem Server betriebsbereit zu haben, bevor der portable Router seinen Router-Betrieb beendet. Hierdurch ist sichergestellt, dass der Datenstrom auf wenigstens einem Weg an den Server übermittelt wird und somit am Terminal zur Verfügung steht.

Es ist auch vorgesehen, das Umschalten durch den Trigger frühestens dann einzuleiten, wenn eine stabile WLAN-Verbindung zwischen dem portablen Router und dem mobilen Router aufgebaut ist. Hierdurch ist sichergestellt, dass es durch ein zu frühes Umschalten nicht zu einer ungewünscht großen Lücke bei der Datenübertragung kommt.

Weiterhin ist es vorgesehen, das Umschalten durch den Trigger erst dann einzuleiten, wenn auch bereits eine aktive Übertragung von Vitaldaten durch den portablen Router über den mobilen Router und das Mobilfunknetz an den Server stattfindet. Hierdurch ist sichergestellt, dass das EKG-Gerät beim Umschalten bereits arbeitet und Vitaldaten liefert, so dass eine ggf. erforderliche Fehlersuche vereinfacht ist.

Erfindungsgemäß ist es vorgesehen, das Umschalten durch den Trigger dann einzuleiten, wenn auch ein entsprechender Befehl des Servers an den portablen Router gesendet wird, wobei dieser Befehl erst ausgeführt wird, wenn wenigstens ein anderer Umschaltvorgang nämlich ein automatisches Umschalten einer am Körper getragenen Kamera auf eine Fahrzeugkamera erfolgt ist. Hierdurch kann indirekt bestätigt werden, dass der Patient bereits in der Nähe des Rettungsfahrzeugs ist oder bereits in das Rettungsfahrzeug verbracht ist, so dass z.B. ein zu frühes Umschalten, bei welchem der portable Router noch nicht dauerhaft zuverlässig in dem zweiten WLAN-Netz eingebucht werden kann, verhindert ist.

Alternativ ist es erfindungsgemäß auch vorgesehen, das Umschalten des Triggers dann einzuleiten, wenn auch eine Kommunikationsverbindung zwischen dem Fahrzeug und dem portablen Router aufgebaut ist, wobei die Kommunikationsverbindung durch Kommunikationsmodule geringer Reichweite wie insbesondere durch Bluetooth-Low-Power-Module hergestellt wird. Auch hierdurch kann indirekt bestätigt werden, dass der Patient bereits in der Nähe des Rettungsfahrzeugs ist oder bereits in das Rettungsfahrzeug verbracht ist, so dass z.B. ein zu frühes Umschalten, bei welchem der portable Router noch nicht dauerhaft zuverlässig in dem zweiten WLAN-Netz eingebucht werden kann, verhindert ist.

Das erfindungsgemäße Telenotarzt-System umfasst einen mobilen Router, einen portablen Router, ein EKG-Gerät oder dergleichen, einen Server, ein Terminal und einen Trigger, wobei das EKG-Gerät drahtlos mit dem portablen Router verbunden ist, wobei der Server und das Terminal miteinander verbunden sind, wobei der portable Router in einem ersten Betriebsmodus in einem Router-Betrieb derart arbeitet, dass über ein Mobilfunknetz eine Verbindung zu dem Server aufgebaut ist, so dass von dem EKG-Gerät erfasste Vitaldaten als kontinuierlicher Datenstrom an dem Terminal abrufbar sind, wobei der portable Router in einem zweiten Betriebsmodus in einem Repeater-Betrieb derart arbeitet, dass über ein WLAN-Netz des mobilen Routers und das Mobilfunknetz eine Verbindung zu dem Server aufgebaut ist, so dass die von dem EKG-Gerät erfassten Vitaldaten als kontinuierlicher Datenstrom an dem Terminal abrufbar sind, wobei der Trigger derart ausgebildet ist, dass dieser den portablen Router von dem ersten Betriebsmodus in den zweiten Betriebsmodus umschaltet, wobei dies abhängig von wenigstens einem von dem Trigger empfangenen Signal und/oder erfassten Messwert erfolgt. Durch eine derartiges Telenotarzt-System, bei welchem das WLAN-fähige EKG-Gerät mittels des portablen Routers wahlweise direkt über ein Mobilfunknetz oder über ein zweites WLAN-Netz mittelbar über ein Mobilfunknetz mit einem Server verbunden ist, lässt es sich vermeiden, dass zu dem WLAN-fähigen EKG-Gerät eine WLAN-Verbindung neu aufgebaut werden muss und hierdurch eine signifikante Unterbrechung der Verbindung zum Server in Kauf genommen werden muss. Diese Unterbrechung wird durch eine Verbindungsübergabe - auch Handover genannt - vermieden, bei welcher der portable Router, mit welchem das WLAN-fähige EKG-Gerät per WLAN verbunden ist, von einem Router-Betrieb in einen Repeater-Betrieb wechselt. Dies ist mit den bestehenden Protokollen und verfügbaren Routern wesentlich schneller möglich und auch besser automatisierbar als ein Abmelden des WLAN-fähigen EKG-Geräts von einem ersten WLAN-Netz und ein nachfolgendes Anmelden des WLAN-fähigen EKG-Geräts an einem zweiten WLAN-Netz.

Erfindungsgemäß ist es vorgesehen, dass der portable Router zur ununterbrochenen Übermittlung des kontinuierlichen Datenstroms zumindest während des Umschaltens in dem ersten Betriebsmodus und in dem zweiten Betriebsmodus arbeitet. Hierdurch ist sichergestellt, dass der Datenstrom auf wenigstens einem Weg an den Server übermittelt wird und somit am Terminal zur Verfügung steht.

Im Sinne der Erfindung wird unter einem EKG-Gerät oder dergleichen ein medizinisches Gerät verstanden, welches kontinuierlich Vitaldaten und/oder mit diesen zusammenhängende Daten erfasst und diese als kontinuierlichen Datenstrom ausgibt.

Im Sinne der Erfindung wird unter einem Trigger eine Umschalteinrichtung verstanden, welche auf der Basis wenigstens eines Signals und/oder Messwerts den portablen Router von einem Router-Betrieb auf einen Repeater-Betrieb umschaltet.

Unter einem Mobilfunknetz ist im Sinne der Erfindung ggf. auch ein Satellitenkommunikationssystem oder jedes andere Datenfunksystem für größere Reichweiten zu verstehen.

Weitere Einzelheiten der Erfindung werden in der Zeichnung anhand eines schematisch dargestellten Ausführungsbeispiels beschrieben.

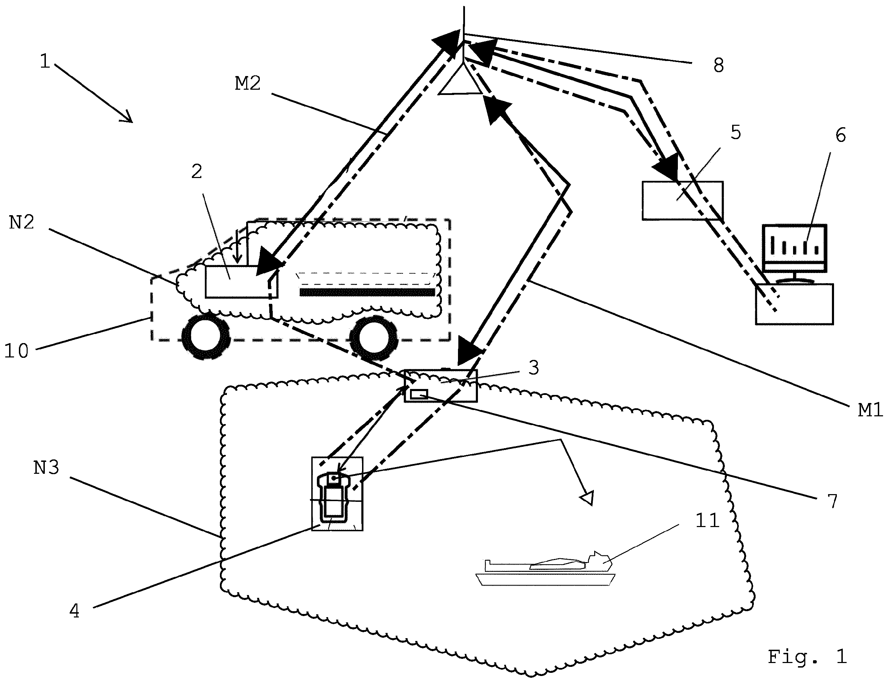

Hierbei zeigt Figur 1 eine schematische Ansicht eines erfindungsgemäßen Telenotarzt-Systems.

In der Figur 1 ist skizzenhaft und schematisch eine Telenotarzt-System 1 dargestellt. Das Telenotarzt-System 1 umfasst einen mobilen Router 2, einen portablen Router 3, ein WLAN-fähiges EKG-Gerät 4, einen Server 5, ein Terminal 6 und einen Trigger 7. Das EKG-Gerät 4 ist per WLAN drahtlos mit dem portablen Router 3 verbunden, wobei die Verbindung in einem ersten WLAN-Netz N3 aufgebaut ist, welches von dem portablen Router 3 bereitgestellt ist. Der Server 5 und das Terminal 6 stehen drahtlos oder drahtgebunden in Kommunikationsverbindung. Der portable Router 3 arbeitet in einem ersten Betriebsmodus M1 derart in einem Router-Betrieb, dass über ein Mobilfunknetz 8 eine Verbindung zu dem Server 5 aufgebaut ist, so dass von dem EKG-Gerät 4 erfasste Vitaldaten als kontinuierlicher Datenstrom an dem Terminal 6 abrufbar sind. In einem zweiten Betriebsmodus M2 arbeitet der portable Router 3 derart, dass über ein zweites WLAN-Netz N2, welches von dem mobilen Routers 2 zur Verfügung gestellt wird, und über das Mobilfunknetz 8, mit welchem der mobile Router 2 Verbindung hat, eine Verbindung zu dem Server 5 aufgebaut ist, so dass von dem EKG-Gerät 4 erfassten Vitaldaten als kontinuierlicher Datenstrom an dem Terminal 5 abrufbar sind. Von dem Trigger 7 wird der portable Router 3 ereignisabhängig von dem ersten Betriebsmodus M1 in den zweiten Betriebsmodus M2 umschaltet. Dies erfolgt abhängig von wenigstens einem von dem Trigger empfangenen Signal und/oder erfassten Messwert. Zur ununterbrochenen Übermittlung des kontinuierlichen Datenstroms, welchen das EKG-Gerät 4 erzeugt, arbeitet der portable Router 3 zumindest während des Umschaltens in dem ersten Betriebsmodus M1 und in dem zweiten Betriebsmodus M2.

Selbstverständlich können der portable Router 2 und der mobile Router 3 auch mit unterschiedlichen Mobilfunknetzen mit dem Server verbunden sein.

Das erfindungsgemäße Verfahren zum Betrieb des Telenotarzt-Systems 1 umfasst die Schritte:
- Einbinden des WLAN-fähigen EKG-Geräts 4 in das von dem portablen Router 3 bereitgestellte erste WLAN-Netz N3;
- Erstversorgung eines Unfallopfers 9 außerhalb eines Fahrzeugs 10, wobei von dem EKG-Gerät 4 erfasste Vitaldaten des Unfallopfers 9 von dem portablen Router 3 in einem Router-Betrieb aus dem ersten WLAN-Netz N3 über ein Mobilfunknetz 8, in welches der portable Router 3 eingebunden ist, an den Server 5 übertragen werden, um diese an dem Terminal 6 bereitzustellen;
- Verbringen des Unfallopfers 9 nach der Erstversorgung zu dem und in das Fahrzeug 10 und hierbei durch einen Trigger 7 veranlasstes Umschalten des portablen Routers 3 von seinem Router-Betrieb auf einen Repeater-Betrieb, wobei sich der portable Router 3 hierzu als Repeater 11 mit dem von dem mobilen Router 2 bereit gestellten zweiten WLAN-Netz N2 verbindet,
- Weiterleiten der von dem EKG-Geräts 4 erfassten Vitaldaten von dem portablen Router 3 in dem Repeater-Betrieb aus dem ersten WLAN-Netz N3 des portablen Routers 3 über das zweite WLAN-Netz N2 des mobilen Routers 2 an den mobilen Router 2 und Weiterleiten der Vitaldaten von dem mobilen Router 2 über ein Mobilfunknetz 8 über eine bestehende Funkverbindung an den Server 5, so dass von dem EKG-Gerät erfasste Vitaldaten in einem kontinuierlichen Datenstrom an dem Terminal bereitgestellt werden.

Optional ist ein Zwischenschritt vorgesehen, bei welchem die Verbindung des EKG-Geräts 2 über den portablen Router 3, den mobilen Router 2 und das Mobilfunknetz 8 zu dem Server 5 betriebsbereit ist bevor der portable Router 3 seinen Router-Betrieb beendet.

### Bezugszeichenliste:

- 1: Telenotarzt-System
- 2: mobiler Router
- 3: portabler Router
- 4: WLAN-fähiges EKG-Gerät
- 5: Server
- 6: Terminal
- 7: Trigger
- 8: Mobilfunknetz
- 9: Umfallopfer
- 10: Fahrzeug
- 11: Repeater

- M1: erster Betriebsmodus (Router-Betrieb)
- M2: zweiter Betriebsmodus (Repeater-Betrieb)
- N3: ersten WLAN-Netz aufgebaut von 3
- N2: zweites WLAN-Netz aufgebaut von 2

## Patentansprüche

1. Verfahren zum Betrieb eines Telenotarzt-Systems (1), welches
- einen in einem Fahrzeug (10) angeordneten mobilen Router (2),
- einen entfernt von dem Fahrzeug (10) einsetzbaren portablen Router(3),
- einen Server (5) und
- ein mit dem Server (5) verbundenes Terminal (6) für einen Notarzt umfasst,
- umfassend die Schritte:
- Einbinden eines WLAN-fähigen EKG-Geräts (4) oder dergleichen in ein von dem portablen Router (3) bereit gestelltes erstes WLAN-Netz (N3);
- Erstversorgung eines Unfallopfers (9) außerhalb des Fahrzeugs (10), wobei von dem EKG-Gerät (4) erfassten Vitaldaten des Unfallopfers (9) von dem portablen Router (3) in einem Router-Betrieb aus dem ersten WLAN-Netz (N3) über ein Mobilfunknetz (8), in welches der portable Router (3) eingebunden ist, an den Server (5) übertragen werden, um diese an dem Terminal (6) bereitzustellen;
- Verbringen des Unfallopfers (9) nach der Erstversorgung zu dem und in das Fahrzeug (10) und hierbei durch einen Trigger (7) gesteuertes Umschalten des portablen Routers (3) von seinem Router-Betrieb auf einen Repeater-Betrieb, wobei sich der portable Router (3) hierzu als Repeater (11) mit einem von dem mobilen Router (2) bereit gestellten zweiten WLAN-Netz (N2) verbindet,
- Weiterleiten der von dem EKG-Gerät (4) erfassten Vitaldaten von dem portablen Router (2) in dem Repeater-Betrieb aus dem ersten WLAN-Netz (N3) des portablen Routers (3) über das zweite WLAN-Netz (N2) des mobilen Routers (2) an den mobilen Router (2) und Weiterleiten der Vitaldaten von dem mobilen Router (2) über ein Mobilfunknetz (8) über eine bestehende Funkverbindung an den Server (5), so dass von dem EKG-Gerät (4) erfasste Vitaldaten in einem kontinuierlichen Datenstrom an dem Terminal (6) bereitgestellt werden,
- wobei der Trigger (7) das Umschalten dann einleitet, wenn auch ein entsprechender Befehl des Servers (5) an den portablen Router (3) gesendet wird, wobei dieser Befehl erst ausgeführt wird, wenn wenigstens ein anderer Umschaltvorgang nämlich ein automatisches Umschalten einer am Körper getragenen Kamera auf eine Fahrzeugkamera erfolgt ist oder
- wobei der Trigger (7) das Umschalten dann einleitet, wenn auch eine Kommunikationsverbindung zwischen dem Fahrzeug (10) und dem portablen Router (3) aufgebaut ist, wobei die Kommunikationsverbindung durch Kommunikationsmodule geringer Reichweite wie insbesondere durch Bluetooth-Low-Power-Module hergestellt wird.

2. Verfahren nach Anspruch 1, **dadurch gekennzeichnet, dass** die Verbindung des portablen Routers (3) über den mobilen Router (2) und das Mobilfunknetz (8) zu dem Server (5) betriebsbereit ist bevor der portable Router (3) seinen Router-Betrieb beendet.

3. Verfahren nach wenigstens einem der vorhergehenden Ansprüche, **dadurch gekennzeichnet, dass** der Trigger (7) das Umschalten frühestens dann einleitet, wenn eine stabile WLAN-Verbindung zwischen dem portablen Router (3) und dem mobilen Router (2) aufgebaut ist.

4. Verfahren nach wenigstens einem der vorhergehenden Ansprüche, **dadurch gekennzeichnet, dass** der Trigger (7) das Umschalten erst dann einleitet, wenn auch bereits eine aktive Übertragung von Vitaldaten durch den portablen Router (3) über den mobilen Router (2) und das Mobilfunknetz (8) an den Server (5) stattfindet.

5. Telenotarzt-System (1) zur Durchführung des Verfahrens nach einem der Ansprüche 1 bis 4, umfassend,
- einen mobilen Router (2),
- einen portablen Router (3),
- ein EKG-Gerät (4) oder dergleichen,
- einen Server (5),
- ein Terminal (6) und
- einen Trigger (7),
- wobei das EKG-Gerät (4) drahtlos mit dem portablen Router (3) verbunden ist,
- wobei der Server (5) und das Terminal (6) miteinander verbunden sind,
- wobei der portable Router (3) in einem ersten Betriebsmodus (M1) in einem Router-Betrieb derart arbeitet, dass über ein Mobilfunknetz (8) eine Verbindung zu dem Server (5) aufgebaut ist, so dass von dem EKG-Gerät (4) erfasste Vitaldaten als kontinuierlicher Datenstrom an dem Terminal (6) abrufbar sind,
- wobei der portable Router (3) in einem zweiten Betriebsmodus (M2) in einem Repeater-Betrieb derart arbeitet, dass über ein WLAN-Netz (N3) des mobilen Routers (3) und das Mobilfunknetz (8) eine Verbindung zu dem Server (5) aufgebaut ist, so dass die von dem EKG-Gerät (4) erfassten Vitaldaten als kontinuierlicher Datenstrom an dem Terminal (6) abrufbar sind,
- wobei der Trigger (7) derart ausgebildet ist, dass dieser den portablen Router (3) von dem ersten Betriebsmodus (M1) in den zweiten Betriebsmodus (M2) umschaltet, wobei dies abhängig von wenigstens einem von dem Trigger (7) empfangenen Signal und/oder erfassten Messwert erfolgt.
- wobei der portable Router (3) zur ununterbrochenen Übermittlung des kontinuierlichen Datenstroms zumindest während des Umschaltens in dem ersten Betriebsmodus (M1) und in dem zweiten Betriebsmodus (M2) arbeitet.

## Claims

1. Method for operating a tele-emergency physician system (1), which comprises
- a mobile router (2) arranged in a vehicle (10),
- a portable router (3) usable remotely from the vehicle (10),
- a server (5), and
- a terminal (6), connected to the server (5), for an emergency physician,
- comprising the following steps:
- integrating a WLAN-capable ECG device (4) or the like into a first WLAN network (N3) provided by the portable router (3);
- providing first aid to an accident victim (9) outside the vehicle (10), wherein vital data of the accident victim (9) acquired by the ECG device (4) are transmitted by the portable router (3) in router operation from the first WLAN network (N3) via a mobile wireless network (8), into which the portable router (3) is integrated, to the server (5), to provide these data at the terminal (6);
- moving the accident victim (9) after the first aid to the and into the vehicle (10) and at the same time switching over the portable router (3), controlled by a trigger (7), from its router operation to repeater operation, wherein the portable router (3) connects itself for this purpose as a repeater (11) to a second WLAN network (N2) provided by the mobile router (2),
- forwarding the vital data acquired by the ECG device (4) by way of the portable router (2) in repeater operation from the first WLAN network (N3) of the portable router (3) via the second WLAN network (N2) of the mobile router (2) to the mobile router (2) and forwarding the vital data by way of the mobile router (2) via a mobile wireless network (8) via an existing radio link to the server (5), so that vital data acquired by the ECG device (4) are provided in a continuous data stream at the terminal (6),
- wherein the trigger (7) initiates the switching over when a corresponding command of the server (5) is also sent to the portable router (3), wherein this command is only executed when at least one other switchover procedure, namely automatically switching over a camera worn on the body to a vehicle camera, has taken place or
- wherein the trigger (7) initiates the switching over when a communication connection is also established between the vehicle (10) and the portable router (3), wherein the communication connection is established by short-range communication modules, for example in particular by Bluetooth low-power modules.

2. Method according to Claim 1, **characterized in that** the connection of the portable router (3) to the server (5) via the mobile router (2) and the mobile wireless network (8) is ready for operation before the portable router (3) ends its router operation.

3. Method according to at least one of the preceding claims, **characterized in that** the trigger (7) initiates the switching over at the earliest when a stable WLAN connection is established between the portable router (3) and the mobile router (2).

4. Method according to at least one of the preceding claims, **characterized in that** the trigger (7) initiates the switching over only when active transmission of vital data by the portable router (3) via the mobile router (2) and the mobile wireless network (8) to the server (5) already takes place.

5. Tele-emergency physician system (1) for carrying out the method according to any one of Claims 1 to 4, comprising
- a mobile router (2),
- a portable router (3),
- an ECG device (4) or the like,
- a server (5),
- a terminal (6), and
- a trigger (7),
- wherein the ECG device (4) is wirelessly connected to the portable router (3),
- wherein the server (5) and the terminal (6) are connected to one another,
- wherein the portable router (3) operates in a first operating mode (M1) in router operation such that a connection to the server (5) is established via a mobile wireless network (8), so that vital data acquired by the ECG device (4) are retrievable as a continuous data stream at the terminal (6),
- wherein the portable router (3) operates in a second operating mode (M2) in repeater operation such that a connection to the server (5) is established via a WLAN network (N3) of the mobile router (3) and the mobile wireless network (8), so that the vital data acquired by the ECG device (4) are retrievable as a continuous data stream at the terminal (6),
- wherein the trigger (7) is designed such that it switches over the portable router (3) from the first operating mode (M1) into the second operating mode (M2), wherein this takes place depending on at least one signal received and/or measured value acquired by the trigger (7),
- wherein the portable router (3) operates at least during the switching over in the first operating mode (M1) and in the second operating mode (M2) for uninterrupted transfer of the continuous data stream.

## Revendications

1. Procédé pour faire fonctionner un système de télémédecine d'urgence (1), lequel comporte
- un routeur mobile (2) disposé dans un véhicule (10),
- un routeur portable (3) utilisable à distance du véhicule (10),
- un serveur (5) et
- un terminal (6) relié au serveur (5) pour un médecin urgentiste,
- comprenant les étapes suivantes :
- intégration d'un appareil d'ECG (4) compatible WLAN ou similaire dans un premier réseau WLAN (N3) mis à disposition par le routeur portable (3) ;
- prodigation des premiers soins à une victime d'accident (9) à l'extérieur du véhicule (10), les données vitales de la victime d'accident (9) acquises par l'appareil d'ECG (4) étant transmises par le routeur portable (3) dans un mode routeur du premier réseau WLAN (N3) au serveur (5) par le biais d'un réseau de radiocommunication mobile (8) auquel le routeur portable (3) est connecté afin de les mettre à disposition sur le terminal (6) ;
- transfert de la victime d'accident (9), après les premiers soins, vers et dans le véhicule (10) et, ce faisant, permutation commandée par un déclencheur (7) du routeur portable (3) de son mode routeur à un mode répéteur, le routeur portable (3), en tant que répéteur (11), se connectant à cet effet à un deuxième réseau WLAN (N2) mis à disposition par le routeur mobile (2),
- retransmission des données vitales acquises par l'appareil d'ECG (4) du routeur portable (2) en mode répéteur depuis le premier réseau WLAN (N3) du routeur portable (3) au routeur mobile (2) par le biais du deuxième réseau WLAN (N2) du routeur mobile (2), puis retransmission des données vitales du routeur mobile (2) au serveur (5) par le biais d'un réseau de radiocommunication mobile (8) par le biais d'une connexion radio existante, de sorte que les données vitales acquises par l'appareil d'ECG (4) sont mises à disposition dans un flux de données continu sur le terminal (6),
- le déclencheur (7) initiant la permutation lorsqu'une instruction correspondante du serveur (5) est également envoyée au routeur portable (3), cette instruction n'étant exécutée que lorsqu'au moins une autre opération de permutation, à savoir une permutation automatique d'une caméra portée sur le corps vers une caméra embarquée dans le véhicule, a eu lieu ou
- le déclencheur (7) initiant la permutation lorsqu'une connexion de communication est également établie entre le véhicule (10) et le routeur portable (3), la connexion de communication étant établie par des modules de communication à courte portée, tels que notamment des modules Bluetooth à basse consommation.

2. Procédé selon la revendication 1, **caractérisé en ce que** la connexion du routeur portable (3) au serveur (5) par le biais du routeur mobile (2) et du réseau de radiocommunication mobile (8) est opérationnelle avant que le routeur portable (3) ne mette fin à son mode routeur.

3. Procédé selon au moins l'une des revendications précédentes, **caractérisé en ce que** le déclencheur (7) initie la permutation au plus tôt lorsqu'une connexion WLAN stable est établie entre le routeur portable (3) et le routeur mobile (2).

4. Procédé selon au moins l'une des revendications précédentes, **caractérisé en ce que** le déclencheur (7) n'initie la permutation que lorsqu'une transmission active de données vitales par le routeur portable (3) vers le serveur (5) a aussi déjà lieu par le biais du routeur mobile (2) et du réseau de radiocommunication mobile (8).

5. Système de télémédecine d'urgence (1) pour la mise en œuvre du procédé selon l'une des revendications 1 à 4, comprenant
- un routeur mobile (2),
- un routeur portable (3),
- un appareil d'ECG (4) ou similaire,
- un serveur (5),
- un terminal (6) et
- un déclencheur (7),
- l'appareil d'ECG (4) étant connecté sans fil au routeur portable (3),
- le serveur (5) et le terminal (6) étant connectés entre eux,
- le routeur portable (3) fonctionnant dans un premier mode de fonctionnement (M1) dans un mode routeur de telle sorte qu'une connexion au serveur (5) est établie par le biais d'un réseau de radiocommunication mobile (8), de sorte que les données vitales acquises par l'appareil d'ECG (4) peuvent être consultées sous forme de flux de données continu sur le terminal (6),
- le routeur portable (3) fonctionnant dans un deuxième mode de fonctionnement (M2) dans un mode répéteur de telle sorte qu'une connexion au serveur (5) est établie par le biais d'un réseau WLAN (N3) du routeur mobile (3) et du réseau de radiocommunication mobile (8), de sorte que les données vitales acquises par l'appareil d'ECG (4) peuvent être consultées sous forme de flux de données continu sur le terminal (6),
- le déclencheur (7) étant configuré de telle sorte qu'il permute le routeur portable (3) du premier mode de fonctionnement (M1) au deuxième mode de fonctionnement (M2), ceci s'effectuant en fonction d'au moins un signal reçu et/ou d'une valeur de mesure acquise par le déclencheur (7),
- le routeur portable (3) fonctionnant en vue d'une communication ininterrompue du flux de données continu au moins pendant la permutation dans le premier mode de fonctionnement (M1) et dans le deuxième mode de fonctionnement (M2).
